# EUROPÄISCHE PATENTSCHRIFT

(11) **EP 1 509 207 B1**
(45) Veröffentlichungstag und Bekanntmachung des Hinweises auf die Patenterteilung: **29.04.2009**
(21) Anmeldenummer: 03730048.0
(22) Anmeldetag: 15.05.2003
(51) Int. Cl.: A61K 9/20, A61K 9/28, A61K 31/55, A61P 25/08, A61P 25/06

(54) **PHARMAZEUTISCHE ZUSAMMENSETZUNG, ENTHALTEND OXCARBAZEPIN MIT KONTROLLIERTER WIRKSTOFFFREISETZUNG**
PHARMACEUTICAL COMPOSITION CONTAINING OXCARBAZEPINE AND HAVING A CONTROLLED ACTIVE SUBSTANCE RELEASE
COMPOSITION PHARMACEUTIQUE À BASE D'OXCARBAZEPINE, À LIBÉRATION RETARD DU PRINCIPE ACTIF

(30) Priorität: 31.05.2002 DE 10224177; 31.05.2002 DE 10224170; 30.10.2002 DE 10250566
(43) Veröffentlichungstag der Anmeldung: 02.03.2005
(73) Patentinhaber: Desitin Arzneimittel GmbH, D-22335 Hamburg (DE)
(72) Erfinder: FRANKE, Hanshermann, 22889 Tangstedt (DE); LENNARTZ, Peter, 22529 Hamburg (DE)
(74) Vertreter: UEXKÜLL & STOLBERG
(86) Internationale Anmeldenummer: PCT/EP2003/005116
(87) Internationale Veröffentlichungsnummer: WO 2003/101430

(56) Entgegenhaltungen:
- WO-A-01/32183
- US-A1- 2002 022 056
- US-B1- 6 296 873
- SHESKEY P ET AL: "Roll compaction granulation of a controlled-release matrix tablet formulation containing HPMC: Effect of process scale-up on robustness of tablets, tablet stability, and predicted in vivo performance" PHARMACEUTICAL TECHNOLOGY 2000 UNITED STATES, Bd. 24, Nr. 11, 2000, Seiten 30-52, XP002254876 ISSN: 0147-8087

## Beschreibung

Die vorliegende Erfindung betrifft pharmazeutische Zusammensetzungen, insbesondere peroral einzunehmende Zusammensetzungen, mit einem wirksamen Gehalt an Oxcarbazepin.

Oxcarbazepin wird zur Behandlung von Krankheiten des epileptischen Formenkreises, zur Bekämpfung von neuralgischen oder zerebo-vaskülaren Schmerzen oder zum Alkoholentzug verwendet. Oxcarbazepin wird im Körper zu Monohydroxydihydrocarbamazepin (MHD) umgesetzt, die die eigentliche Wirkkomponente ist.

Heute in der Therapie eingesetzte Zusammensetzungen zur peroralen Verabreichung von Oxcarbazepin liegen ausschließlich in Form von nichtretardierenden peroralen Darreichungsformen vor. Diese bewirken nach einmaliger Verabreichung *in vivo* den schnellen Aufbau der Plasmaspiegel von Oxcarbazepin und MHD. Nach beendeter Resorption kommt es dann zu einem relativ schnellen Abfall der Plasmakonzentration der Wirkstoffe.

Der schnelle Wirkstoffanstieg konventioneller Zusammensetzungen ist mit teilweise erheblichen Nebenwirkungen verbunden. Das Auftreten von Plasmaspitzen kann insbesondere bei Verabreichung von Oxcarbazepin zu starken Beeinträchtigungen des Allgemeinbefindens wie Übelkeit und Schwindel bis hin zur Ohnmacht führen. Um dies zu vermeiden, muß der Patient zwei- bis dreimal täglich eine oder mehrere Tabletten einnehmen. Nur so kann ein ausreichend gleichmäßiger Verlauf des Wirkstoffspiegels im Plasma erreicht werden.

Zwischen dem Grad der Einhaltung der vorgegebenen Arzneimitteleinnahme über den Tag und der Häufigkeit der Einnahme pro Behandlungstag besteht jedoch ein umgekehrt proportionaler Zusammenhang: je mehr Einnahmen pro Tag (hohe Einnahmefrequenz) desto niedriger ist auf Dauer gesehen das Maß der Einhaltung des notwendigen Einnahmeschemas (niedrige "Compliance"). Ursachen hierfür sind neben dem z.B. simplen Vergessen eines Einnahmezeitpunktes, auch die Unwilligkeit der Patienten Medikamente in ungünstigen Situationen einzunehmen. Zu diesen Situationen zählen typischerweise z.B. gemeinsame Mahlzeiten, berufliche Besprechungen oder in Gruppen gehaltene Veranstaltungen. Dies gilt in besonders hohem Maße für Epilepsie-Patienten, da diese Krankheit noch heute mit einem gesellschaftlichen Stigma belegt ist.

Z.B. beschzeibt WO 01 32 183 A Oxcorbazebinzusammensetzumgen, die zwei mal taglich verabzeicht werden müssen.

Aufgabe der vorliegenden Erfindung ist es daher, pharmazeutische Zusammensetzungen zur peroralen Verabreichung bereitzustellen, die die vorgenannten Nachteile nicht aufweisen, indem sie bei einmaliger täglicher Einnahme zu einem sich in geeigneter Geschwindigkeit aufbauenden, langanhaltenden Wirkstoffspiegel des Metaboliten MHD im Plasma führen. Dabei dürfen minimal wirksame Plasmaspiegel (subtherapeutische Plasmaspiegel) nicht unterschritten werden. Weiterhin sollten sogenannte Plasmaspitzen, insbesondere während der Anflutungsphase, soweit wie möglich vermieden werden.

Ferner ist es eine Aufgabe der vorliegenden Erfindung, ein Verfahren zur Herstellung solcher Zusammensetzungen bereitzustellen.

Die Aufgaben werden durch eine pharmazeutische Zusammensetzung gemäß Anspruch 1 und einem Verfahren gemäß Anspruch 6 gelöst. Überraschenderweise wurde gefunden, dass Zusammensetzungen, die *in-vitro* gemäß dem USP Paddle-Verfahren (USP 24, Methode 724, App. 2 - in 1 L 2 Gew.-%iger Natriumdodecylsulfat-Lösung als Freisetzungsmedium, bei einer Rührgeschwindigkeit von 75 U/min) folgende Mengen Oxcarbazepin freisetzen
15 min: 55 bis 85%
30 min: 75 bis 95%
45 min: 85 bis 100%
60 min: 90 bis 100%
zu sich einem langsam aufbauenden, langanhaltenden Wirkstoffspiegel des Metaboliten MHD im Plasma führen.

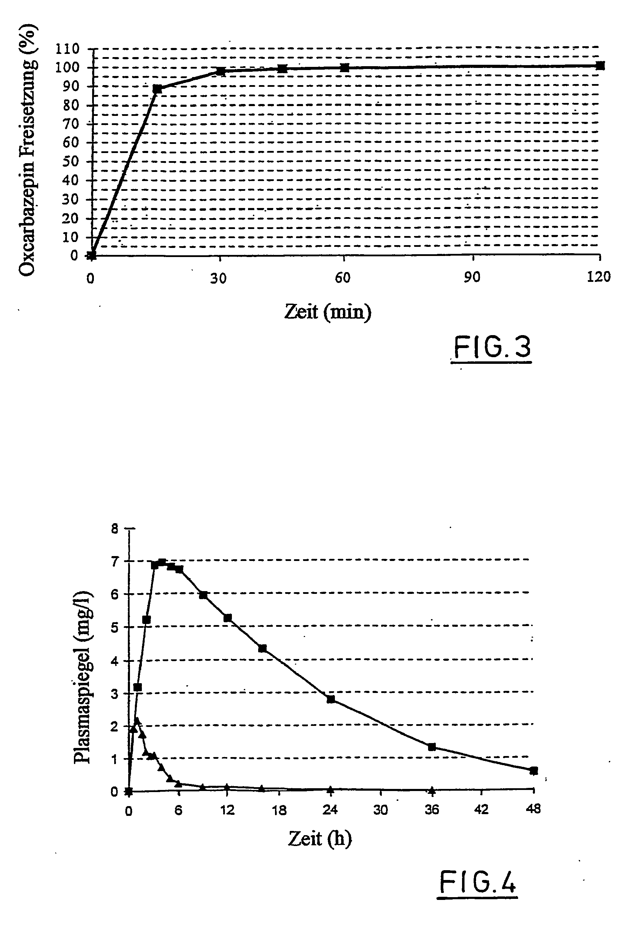

Handelsübliche Tabletten setzen gemäß der gleichen Freisetzungsmethode dagegen folgende Mengen Oxcarbazepin frei (siehe Fig. 3) :
15 min: etwa 88 bis 90%
30 min: etwa 95 bis 100%
45 min: etwa 98 bis 100%
60 min: etwa 100%
und weisen die oben genannten Nachteile auf.

Das Ergebnis ist deshalb überraschend, weil der *in-vitro*-Freisetzungsverlauf von Oxcarbazepin der erfindungsgemäßen Zusammensetzungen nur unwesentlich unterhalb desjenigen von handelsüblichen Tabletten liegt, bei dem üblicherweise keine ausreichende Wirkungsverlängerung erwartet wird. Typische Retard-Formulierungen, mit niedrigem folgendem *in-vitro*-Freisetzungsprofil (60 min: etwa 40% Oxcarbazepin-Freisetzung) haben sich dagegen als unwirksam erwiesen.

Vorzugsweise setzen die erfindungsgemäßen Zusammenstzungen *in-vitro* gemäß dem USP Paddle-Verfahren (USP 24, Methode 724, App. 2 in 1 L 2 Gew.-%iger Natriumdodecylsulfat-Lösung als Freisetzungsmedium, bei einer Rührgeschwindigkeit von 75 U/min) folgende Mengen Oxcarbazepin frei:
15 min: 65 bis 80%
30 min: 85 bis 95%
45 min: 90 bis 100%
60 min: 95 bis 100%.

Nach peroraler Aufnahme der erfindungsgemäßen Zusammensetzung, enthaltend 600 mg Oxcarbazepin, werden vorzugsweise folgende Plasmakonzentrationen an Oxcarbazepin:

| | |
|---|---|
| 1,5 bis 2 Stunden | 0,2 bis 0,6 mg/L |
| 5,5 bis 6,5 Stunden | 0,1 bis 0,3 mg/L |
| 11 bis 13 Stunden | 0,1 bis 0,2 mg/L |
| 23 bis 25 Stunden | 0,0 bis 0,2 mg/L |

und folgende Plasmakonzentrationen an MHD erreicht:

| | |
|---|---|
| 1,5 bis 2 Stunden | 1 bis 4 mg/L |
| 5,5 bis 6,5 Stunden | 3 bis 5 mg/L |
| 11 bis 13 Stunden | 3 bis 5 mg/L |
| 23 bis 25 Stunden | 2,5 bis 4,5 mg/L. |

Nach peroraler Aufnahme der erfindungsgemäßen Zusammensetzung, enthaltend 600 mg Oxcarbazepin, werden besonders bevorzugt folgende Plasmakonzentrationen an Oxcarbazepin:

| | |
|---|---|
| 1,5 bis 2 Stunden | 0,3 bis 0,5 mg/L |
| 5,5 bis 6,5 Stunden | 0,1 bis 0,4 mg/L |
| 11 bis 13 Stunden | 0,1 bis 0,2 mg/L |
| 23 bis 25 Stunden | 0,0 bis 0,1 mg/L |

und folgende Plasmakonzentrationen an MHD erreicht:

| | |
|---|---|
| 1,5 bis 2 Stunden | 1 bis 3 mg/L |
| 5,5 bis 6,5 Stunden | 3,5 bis 4,5 mg/L |
| 11 bis 13 Stunden | 3,5 bis 4,5 mg/L |
| 23 bis 25 Stunden | 2,5 bis 4 mg/L. |

Vorzugsweise ergibt die erfindungsgemäße pharmazeutische Zusammensetzung in-vivo nach peroraler Aufnahme der Zusammensetzung, enthaltend 600 mg Oxcarbazepin, im Zeitraum von 4 Stunden nach der Aufnahme bis 21 Stunden nach der Aufnahme einen mittleren Plasmaspiegel an MHD von 3 bis 5 mg/mL und einen maximalen Plasmaspiegel (cₘₐₓ) an MHD von 3 bis 5 mg/mL.

Die erfindungsgemäßen Zusammensetzungen können hergestellt werden, indem eine Mischung, die, bezogen auf deren Gesamtgewicht,
A. 60 bis 95 Gew.-% Oxcarbazepin,
B. 3 bis 30 Gew.-% mikrokristalline Cellulose,
C. 1 bis 20 Gew.-% Ammoniummethacylat-Copolymer und/oder Polymethacrylsäure Polymer,
D. 0,05 bis 4 Gew.-% Zerfallhilfsmittel und
E. Farbstoff
enthält, hergestellt und anschließend kompaktiert wird. Vorzugsweise enthält die Mischung, bezogen auf deren Gesamtgewicht:
A.80 bis 90 Gew.-%, Oxcarbazepin,
B. 5 bis 15 Gew.-% mikrokristalline Cellulose,
C. 2 bis 10 Gew.-% Ammoniummethacylat-Copolymer und/oder Polymethacrylsäure Polymer,
D. 0,1 bis 2 Gew.-% Zerfallhilfsmittel und
E. Farbstoff.

Geeignete Zerfallhilfsmittel sind insbesondere Natrium-Carboxymethylstärke, Croscarmellose-Natrium und Polyvinyl-Polypyrrolidon.

Der Einsatz von Farbstoffen ist bei Oxcarbazepin Präparaten auf Grund der möglichen Bildung von gefärbten Abbauprodukten üblich. Bei der erfindungsgemäßen Zusammensetzung werden keine der häufig als Farbstoff eingesetzten Eisenoxide/Eisenhydroxide verwendet, da diese die Bildung von Abbauprodukten aus Oxcarbazepin begünstigen können. Weiterhin kann die resultierende Eisen-Aufnahme bei hochdosierten Arzneistoffen wie Oxcarbazepin toxikologisch bedenklich werden. Als Farbstoffe können organische Verbindungen und Farblacke aus organischen Verbindungen eingesetzt werden. Insbesondere geeignet sind Riboflavin und Gelborange S Farblack.

Das so erhaltene Kompaktat besitzt sehr gute Fließeigenschaften und benötigt daher keinen weiteren Zusatz eines Fleißregulierungsmittels, wie Kolloidale Kieselsäure (z.B. Aerosil 200^{®}). Insbesondere Kolloidale Kieselsäure kann die Bildung von unerwünschten Abbauprodukten aus Oxcarbazepin bewirken.

Das so erhaltene Kompaktat kann anschließend klassiert und in Hartgelatinekapseln abgefüllt oder kleine Beutelchen (Sachets) abgepackt werden. Vorzugsweise werden jedoch aus dem Kompaktat Tabletten hergestellt, indem zu diesem zunächst, bezogen auf 100 Gew.-Teile des Kompaktats,
F. 0,2 bis 5 Gew.-Teile Tablettenschmiermittel und
G. 10 bis 50 Gew.-Teile mikrokristalline Cellulose
zugemischt werden und das so erhaltene Gemisch zu einer Tablette weiterverarbeitet wird.

Als Tablettenschmiermittel können insbesondere Magnesiumstearat und Calciumstearat eingesetzt werden.

Die so erhaltenen Tabletten können in einem Trommelcoater, unter Einsatz von Wasser und, bezogen aus 100 Gew.-Teile des Kompaktats, von
F. 0,5 bis 10 Gew.-Teilen Polymethacrylsäure-Copolymer
G. 0,025 bis 2 Gew.-Teilen Weichmacher
H. 0,025 bis 2 Gew.-Teilen Trennmittel
I. Farbstoffen und Pigmenten q.s.
befilmt werden.

Die so erhaltenen Tabletten können auch in einem Trommelcoater, unter Einsatz von Wasser und, bezogen aus 100 Gew.-Teile des Kompaktats, von
F. 0,5 bis 10 Gew.-Teilen Filmbildner
G. 0,0 bis 2 Gew.-Teilen Weichmacher
H. 0,005 bis 2 Gew.-Teilen Trennmittel
I. Farbstoffen und Pigmenten q.s.
befilmt werden.

Als Filmbildner können insbesondere eingesetzt werden: Cellulosederivate oder Polyacrylsäurederivate.

Als Weichmacher können insbesondere eingesetzt werden: Triethylcitrat, Triacetin.

Als Trennmittel können insbesondere eingesetzt werden: Talkum, Glycerolmonostearat.

Das Kompaktat kann auch in der Wirbelschicht oder im Schnellmischer unter Zusatz von Wasser befilmt werden, unter Einsatz, bezogen auf 100 Gew.-Teile des Kompaktats, von
F. 0,5 bis 10 Gew.-Teilen Polymetharcylsäure-Copolymer
G. 0,025 bis 2 Gew.-Teilen Weichmacher
H. 0,025 bis 2 Gew.-Teilen Trennmittel

Als Weichmacher und Trennmittel können die jeweils oben genannten Verbindungen eingesetzt werden.

Man erhält ein Granulat, dass anschließend klassiert und in Hartgelatinekapseln abgefüllt oder kleine Beutelchen (Sachets) abgepackt werden kann. Vorzugsweise werden jedoch aus dem Granulat Tabletten hergestellt, indem zu diesem zunächst, bezogen auf 100 Gew.-Teile des Granulates,
I. 0,2 bis 0,5 Gew.-Teile Tablettenschiermittel und
J. 10 bis 50 Gew.-Teile mikrokristalline Cellulose
zugemischt werden und das so erhaltene Gemisch zu einer Tablette weiterverarbeitet wird.

Als Tablettenschmiermittel können insbesondere Magnesiumstearat und Calciumstearat eingesetzt werden.

Die erfindungsgemäßen Zusammensetzungen können auch hergestellt werden, indem ein Granulat, das, bezogen auf dessen Gesamtgewicht,
A. 60 bis 95 Gew.-% Oxcarbazepin
B. 3 bis 30 Gew.-% mikrokristalline Cellulose,
C. 0,05 bis 4 Gew.-% Zerfallshilfsmittel
D. 1 bis 20 Gew.-% Polymer
E. 0,2 bis 5 Gew.-% Weichmacher
F. 0 bis 5 Gew.-% Trennmittel
G. Farbstoff
enhält, in der Wirbelschicht oder im Schnellmischer unter Zusatz von Wasser hergestellt wird. Vorzugsweise enthält das Granulat, bezogen auf dessen Gesamtgewicht:
A. 80 bis 90 Gew.-% Oxcarbazepin
B. 5 bis 15 Gew.-% mikrokristalline Celluslose
C. 0,1 Bis 2 Gew.-% Zerfallshilfsmittel
D. 2 bis 10 Gew.-% Polymer
E. 0,4 bis 2,5 Gew.-% Weichmacher
F. 0 bis 0,25 Gew.-% Trennmittel
G. Farbstoff

Als Polymer können insbesondere eingesetzt werden: Polymethacrylsäureester, Ammoniummethacrylat-Copolymer.

Als Weichmacher und Trennmittel werden vorzugsweise die in Bezug auf die Herstellung von Tabletten vorgenannten Verbindungen eingesetzt.

Als Zerfallshilfsmittel können insbesondere folgende Substanzen eingesetzt werden: Natrium-Carboxymethylstärke, Croscarmellose-Natrium und Polyvinyl-Polypryrrolidon.

Als Farbstoffe können insbesondere organische Farbstoffe und organische Farblacke eingesetzt werden.

Das so erhaltene Granulat kann anschließend in Hartgelatinekapseln abgefüllt oder in kleine Beutelchen (Sachets) abgepackt werden. Vorzugsweise werden jedoch aus dem Granulat Tabletten hergestellt, indem zu diesem zunächst, bezogen auf 100 Gew.-Teile des Granulates,
H. 0,2 bis 0,5 Gew.-Teile Tablettenschmiermittel und
I. 10 bis 50 Gew.-Teile mikrokristalline Cellulose
zugemischt werden und das so erhaltene Gemisch zu einer Tablette weiterverarbeitet wird.

Als Tablettenschmiermittel können wiederum insbesondere Magnesiumstearat und Calciumstearat eingesetzt werden.

Die erfindungsgemäßen pharmazeutischen Zusammensetzungen können vorteilhaft zur Herstellung eines Arzneimittels zur Prophylaxe gegen oder Behandlung von primär generalisierten tonisch-klonischen Anfällen und/oder fokalen Anfällen mit oder ohne sekundäre Generalisierung verwendet werden.

### Beispiele:

### Beispiel 1: Herstellung eines Kompaktats

30 kg Oxcarbazepin wurden mit 2 kg Ammoniummethacrylat-Copolymer (Eudragit RSPOR), 4 kg mikrokristalliner Cellulose und 0,4 kg Natrium-Carboxymethylstärke in einem Schnellmischer 5 Minuten lang gemischt. Die entstandene Mischung wurde auf einem Kompaktor (3-W-Polygrane der Fa. Gerteis Maschinen + Processengineering AG, Jona, Schweiz) kompaktiert. Die entstandene Schülpe wurde mittels Zwangssiebung zerkleinert und das entstandene Kompaktat über ein Rüttelsieb klassiert (1 mm Siebeinsatz, Rüttelsieb der Fa. Engelsmann, Siebrinne mit 0,25 mm Siebeinsatz).

Ein Teil des klassierten Kompaktats wurde auf einer Kapselfüllmaschine in Hartgelatinekapseln Größe 3, 2, 1 und 0 abgefüllt. Dadurch entstanden Dosierungen von 150 bis 300 mg Oxcarbazepin pro Einzeldosis.

Ein weiterer Teil des klassierten kompaktats wurde auf einer Beutelmaschine in kleine Beutelchen (Sachets) abgepackt. Dadurch entstanden Dosierungen von 50 bis 2400 mg Oxcarbazepin pro Einzeldosis.

### Beispiel 2: Herstellung von Tabletten

Es wurde ein Kompaktat gemäß Beispiel 1 hergestellt und klassiert. Das Kompaktat wurde mit 0,5 kg Magnesiumstearat und 8 kg mikrokristalliner Cellulose gemischt und anschließend zu Tabletten gepreßt, wobei Dosierungen zwischen 150 und 600 mg Oxcarbazepin pro Tablette entstanden.

### Beispiel 3: Untersuchung des Freisetzungsverlaufs in vitro

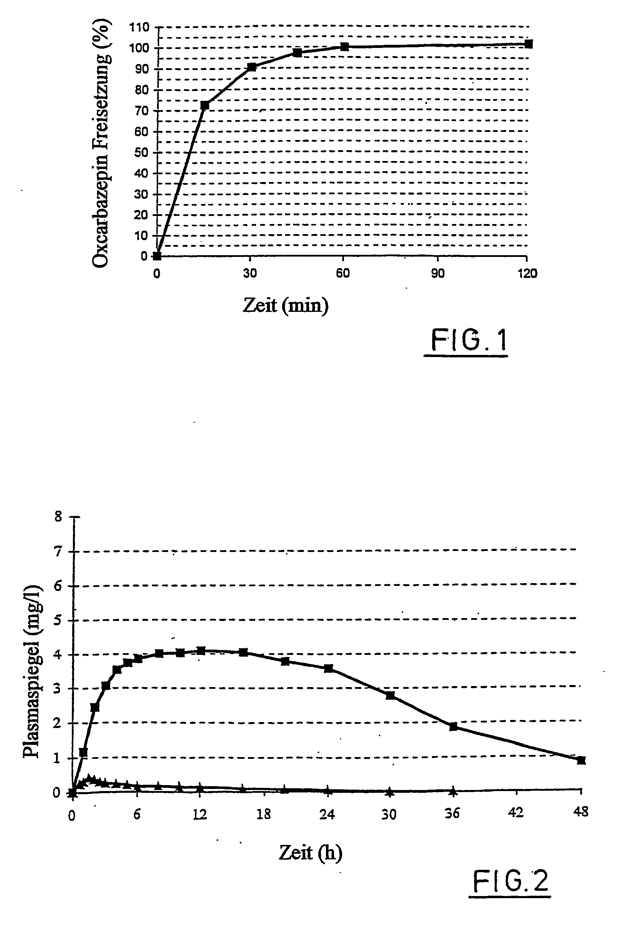

Eine gemäß Beispiel 2 hergestellte Tablette enthaltend 600 mg Oxcarbazepin wurde *in-vitro* gemäß dem USP Paddle-Verfahren (USP 24, Methode 724, App. 2 - in 1 L 2 Gew.-%iger Natriumdodecylsulfat-Lösung als Freisetzungsmedium, bei einer Rührgeschwindigkeit von 75 U/min) untersucht und der Freisetzungsverlauf mit der handelsüblichen Tablette (Trileptale der Fa. Novartis) verglichen. Der Freisetzungsverlauf der erfindungsgemäßen Tablette ist in Fig. 1 und der Freisetzungsverlauf der Vergleichstablette ist in Fig. 3 wiedergegeben. Es zeigt sich, dass die Freisetzung von Oxcarbazepin *in vitro* nur geringfügig langsamer verläuft.

### Beispiel 4: Untersuchung des Plasmaspiegels

Es wurden gemäß Beispiel 2 hergestellte Tabletten enthaltend 600 mg Oxcarbazepin Testpersonen verabreicht und der Plasmaspiegelverlauf von Oxcarbazepin und MHD aufgezeichnet. Die Ergebnisse der Untersuchungen (arithmetische Mittelwerte) sind in Fig. 2 wiedergegeben. Darin kennzeichnen die geschlossenen Dreiecke die Werte für Oxcarbazepin und die geschlossenen Quadrate die Werte für MHD.

Zum Vergleich wurden handelsübliche 600 mg Oxcarbazepin enthaltende Tabletten (Trileptale der Fa. Novartis) verabreicht und der Plasmaspiegelverlauf von Oxcarbazepin und MHD aufgezeichnet. Die Ergebnisse der Untersuchungen (arithmetische Mittelwerte) sind in Fig. 4 wiedergegeben. Darin kennzeichnen die geschlossenen Dreiecke die Werte für Oxcarbazepin und die geschlossenen Quadrate die Werte für MHD.

Die Figuren zeigen, dass der MHD-Plasmaspiegel der erfindungsgemäßen Zusammensetzungen langsam auf eine maximale Konzentration von etwa 3 bis 5 mg/L ansteigt und über einen Zeitraum von etwa 4 Stunden nach Einnahme bis 24 Stunden nach Einnahme in etwa konstant bleibt. Der MHD-Plasmaspiegel der Vergleichszusammensetzungen steigt hingegen rasch auf einen Wert von etwa 7 mg/L an und fällt danach rasch wieder ab.

## Patentansprüche

1. Pharmazeutische Zusammensetzung, enthaltend Oxcarbazepin, die *in-vitro* gemäß dem USP Paddle-Verfahren (USP 24, Methode 724, App. 2 in 1 L 2 Gew.-%iger Natriumdodecylsulfat-Lösung als Freisetzungsmedium, bei einer Rührgeschwindigkeit von 75 U/min) folgende Mengen Oxcarbazepin freisetzt:
15 min: 55 bis 85%
30 min: 75 bis 95%
45 min: 85 bis 100%
60 min: 90 bis 100%.

2. Pharmazeutische Zusammensetzung nach Anspruch 1, enthaltend Oxcarbazepin, die *in-vitro* gemäß dem USP Paddle-Verfahren (USP 24, Methode 724, App. 2 in 1 L 2 Gew.-%iger Natriumdodecylsulfat-Lösung als Freisetzungsmedium, bei einer Rührgeschwindigkeit von 75 U/min) folgende Mengen Oxcarbazepin freisetzt:
15 min: 65 bis 80%
30 min: 85 bis 95%
45 min: 90 bis 100%
60 min: 95 bis 100%.

3. Verfahren zur Herstellung einer pharmazeutischen Zusammensetzung gemäß einem der vorhergehenden Ansprüche, bei dem eine Mischung, die, bezogen auf deren Gesamtgewicht,
a. 60 bis 95 Gew.-% Oxcarbazepin,
b. 3 bis 30 Gew.-% mikrokristalline Cellulose,
c. 1 bis 20 Gew.-% Ammoniummethacylat-Copolymer und/oder Polymethacrylsäure Polymer,
d. 0,05 bis 4 Gew.-% Zerfallshilfsmittel und
e. Farbstoff
enthält, hergestellt und anschließend kompaktiert wird.

4. Verfahren nach Anspruch 3, bei dem eine Mischung, die, bezogen auf deren Gesamtgewicht,
e. 80 bis 90 Gew.-%, Oxcarbazepin,
f. 5 bis 15 Gew.-% mikrokristalline Cellulose,
g. 2 bis 10 Gew.-% Ammoniummethacylat-Copolymer und/oder Polymethacrylsäure Polymer,
h. 0,1 bis 2 Gew.-% Zerfallshilfsmittel und
i. Farbstoff
enthält, hergestellt und anschließend kompaktiert wird.

5. Verfahren nach einem der Ansprüche 3 oder 4, bei dem das Kompaktat gesiebt und unverändert oder ggf. mit Hilfsstoffen versehen in. Kapseln oder in Beutel abgefüllt wird.

6. Verfahren nach einem der Ansprüche 3 oder 4, bei dem nach dem Kompaktieren, bezogen auf 100 Gew.-Teile des Kompaktats,
f. 0,2 bis 5 Gew.-Teile Magnesiumstearat und
g. 10 bis 50 Gew.-Teile mikrokristalline Cellulose
zugemischt werden und das so erhaltene Gemisch zu einer Tablette weiterverarbeitet wird.

7. Verfahren zur Herstellung einer pharmazeutischen Zusammensetzung gemäß einem der Ansprüche 1-2, bei dem ein Granulat, das, bezogen auf dessen Gesamtgewicht,
A. 60 bis 95 Gew.-% Oxcarbazepin
B. 3 bis 30 Gew.-% mikrokristalline Cellulose
C. 0,05 bis 4 Gew.-% Zerfallshilfsmittel
D. 1 bis 20 Gew.-% Polymer
E. 0,2 bis 5 Gew.-% Weichmacher
F. 0 bis 5 Gew.-% Trennmittel
G. Farbstoff
enthält, in der Wirbelschicht oder im Schnellmischer unter Zusatz von Wasser hergestellt wird.

8. Verfahren nach Anspruch 5, bei dem das Granulat, bezogen auf dessen Gesamtgewicht:
A. 80 bis 90 Gew.-% Oxcarbazepin
B. 5 bis 15 Gew.-% mikrokristalline Cellulose
C. 0,1 bis 2 Gew.-% Zerfallshilfsmittel
D. 2 bis 10 Gew.-% Polymer
E. 0,4 bis 2,5 Gew.-% Weichmacher
F. 0 bis 2,5 Gew.-% Trennmittel
G. Farbstoff q.s.
enthält.

9. Verfahren nach einem der Ansprüche 7 oder 8, bei dem, bezogen auf 100 Gew.-Teile des Granulats,
H. 0,2 bis 0,5 Gew.-Teile Tablettenschmiermittel und
I. 10 bis 50 Gew.-Teile mikrokristalline Cellulose
zugemischt werden und das so erhaltene Gemisch zu einer Tablette weiterverarbeitet wird.

10. Verfahren nach einem der Ansprüche 3 oder 4, bei dem das Kompaktat unter Einsatz bezogen auf 100 Gew.-Teile des Kompaktats, von
F. 0,5 bis 10 Gew.-Teilen Polymethacrylsäure Copolymer
G. 0,025 bis 2 Gew.-Teilen Weichmacher
H. 0,025 bis 2 Gew.-Teilen Trennmittel.
im Schnellmischer unter Zusatz von Wasser befilmt wird.

11. Verfahren nach Anspruch 10, bei dem bezogen auf 100 Gew.-Teile des befilmten Kompaktats,
I. 0,2 bis 0,5 Gew.Teile Tabletenschmiermittel und
J. 10 bis 50 Gew.-Teile mikrokristalline Cellulose
zugemischt werden und das so erhaltene Gemisch zu einer Tablette weiterverarbeitet wird.

12. Verfahren nach Anspruch 6, bei dem die Tabletten in einem Trommelcoater, unter Einsatz von Wasser und, bezogen auf 100 Gew.-Teile der Tablette, von
H. 0,5 bis 10 Gew.-Teilen Polymethacrylsäure Copolymer
I. 0,025 bis 2 Gew.-Teilen Weichmacher
J. 0,025 bis 2 Gew.-Teilen Trennmittel und
K. Farbstoff und/oder Pigmenten
befilmt werden.

13. Verfahren nach Anspruch 6, bei dem die Tabletten in einem Trommelcoater, unter Einsatz von Wasser und, bezogen auf 100 Gew.-Teile der Tablette, von
H. 0,5 bis 10 Gew.-Teilen Polymethacrylsäure Copolymer
I. 0,0 bis 2 Gew.-Teilen Weichmacher
J. 0,005 bis 2 Gew.-Teilen Trennmittel und
K. Farbstoff und/oder Pigmenten
befilmt werden.

14. Pharmazeutische Zusammensetzung erhältlich nach dem Verfahren gemäß einem der Ansprüche 4 bis 13.

15. Verwendung einer pharmazeutischen Zusammensetzung gemäß einer der Ansprüche 1 bis 2 und 14 zur Herstellung eines Arzneimittels zur Prophylaxe gegen oder Behandlung von primär generalisierten tonisch-klonischen Anfällen und/oder fokalen Anfällen mit oder ohne sekundäre Generalisierung.

16. Verwendung einer pharmazeutischen Zusammensetzung gemäß einem der Ansprüche 1 bis 2 und 14 zur Herstellung eines Arzneimittels zur Prophylaxe gegen oder Behandlung von neuralgischen und zerebro-vaskulären Schmerzen oder zum Alkoholentzug.

17. Verwendung einer pharmazeutischen Zusammensetzung gemäß einer der Ansprüche 1 bis 2 und 14 zur Herstellung eines Arzneimittels zur einmal täglichen Einnahme.

18. Verwendung einer pharmazeutischen Zusammensetzung gemäß einem der Ansprüche 1 bis 2 und 14 zur Herstellung eines Arzneimittels zur einmal täglichen Einnahme zur Prophylaxe gegen oder Behandlung von neuralgischen und zerebro-vaskulären Schmerzen oder zum Alkoholentzug.

19. Verwendung einer pharmazeutischen Zusammensetzung gemäß einem der Ansprüche 1 bis 2 und 14 zur Herstellung eines Arzneimittels zur einmal täglichen Einnahme zur Prophylaxe gegen oder Behandlung von primär generalisierten tonisch-klonischen Anfällen und/oder fokalen Anfällen mit oder ohne sekundäre Generalisierung.

## Claims

1. Pharmaceutical composition, containing oxcarbazepine, which releases the following quantities of oxcarbazepine:
15 min: 55 to 85%
30 min: 75 to 95%
45 min: 85 to 100%
60 min: 90 to 100%
*in vitro* according to the USP paddle method (USP 24, method 724, app. 2 in 1 L 2 wt.-% sodium dodecylsulphate solution as release medium, at a stirring speed of 75 rpm).

2. Pharmaceutical composition according to claim 1, containing oxcarbazepine, which releases the following quantities of oxcarbazepine:
15 min: 65 to 80%
30 min: 85 to 95%
45 min: 90 to 100%
60 min: 95 to 100%
*in vitro* according to the USP paddle method (USP 24, method 724, app. 2 in 1 L 2 wt.-% sodium dodecylsulphate solution as release medium, at a stirring speed of 75 rpm).

3. Process for the preparation of a pharmaceutical composition according to one of the preceding claims, in which a mixture which, relative to its total weight, contains
a. 60 to 95 wt.-% oxcarbazepine,
b. 3 to 30 wt.-% microcrystalline cellulose,
c. 1 to 20 wt.-% ammonium methacrylate copolymer and/or polymethacrylic acid polymer,
d. 0.05 to 4 wt.-% disintegrant and
e. dye
is prepared and then compacted.

4. Process according to claim 3, in which a mixture which, relative to its total weight, contains
a. 80 to 90 wt.-% oxcarbazepine,
b. 5 to 15 wt.-% microcrystalline cellulose,
c. 2 to 10 wt.-% ammonium methacrylate copolymer and/or polymethacrylic acid polymer,
d. 0.1 to 2 wt.-% disintegrant and
e. dye is prepared and then compacted.

5. Process according to one of claims 3 or 4, in which the compacted material is screened and packed into capsules or into pouches unchanged or optionally provided with excipients.

6. Process according to one of claims 3 or 4, in which after the compacting, relative to 100 parts by weight of the compacted material,
f. 0.2 to 5 parts by weight magnesium stearate and
g. 10 to 50 parts by weight microcrystalline cellulose
are added and the thus-obtained mixture is further processed into a tablet.

7. Process for the preparation of a pharmaceutical composition according to one of claims 1-2, in which a granulated material which, relative to its total weight, contains
A. 60 to 95 wt.-% oxcarbazepine
B. 3 to 30 wt.-% microcrystalline cellulose
C. 0.05 to 4 wt.-% disintegrant
D. 1 to 20 wt.-% polymer
E. 0.2 to 5 wt.-% plasticizer
F. 0 to 5 wt.-% anti-adherent agent
G. dye
is prepared in the fluidised bed or in the high-shear mixer with the addition of water.

8. Process according to claim 5, in which the granulated material, relative to its total weight, contains:
A. 80 to 90 wt.-% oxcarbazepine
B. 5 to 15 wt.-% microcrystalline cellulose
C. 0.1 to 2 wt.-% disintegrant
D. 2 to 10 wt.-% polymer
E. 0.4 to 2.5 wt.-% plasticizer
F. 0 to 2.5 wt.-% anti-adherent agent
G. dye q.s.

9. Process according to one of claims 7 or 8, in which, relative to 100 parts by weight of the granulated material,
H. 0.2 to 0.5 parts by weight tablet lubricant and
I. 10 to 50 parts by weight microcrystalline cellulose are added and the thus-obtained mixture is further processed into a tablet.

10. Process according to one of claims 3 or 4, in which the compacted material, using relative to 100 parts by weight of the compacted material,
F. 0.5 to 10 parts by weight polymethacrylic acid copolymer
G. 0.025 to 2 parts by weight plasticizer
H. 0.025 to 2 parts by weight anti-adherent agent
is coated with a film in the high-shear mixer with the addition of water.

11. Process according to claim 10, in which, relative to 100 parts by weight of the film-coated compacted material,
I. 0.2 to 0.5 parts by weight tablet lubricant and
J. 10 to 50 parts by weight microcrystalline cellulose
are added and the thus-obtained mixture is further processed into a tablet.

12. Process according to claim 6, in which the tablets are coated with a film in a drum coater, using water and, relative to 100 parts by weight of the tablet,
H. 0.5 to 10 parts by weight polymethacrylic acid copolymer
I. 0.025 to 2 parts by weight plasticizer
J. 0.025 to 2 parts by weight anti-adherent agent and
K. dye and/or pigments.

13. Process according to claim 6, in which the tablets are coated with a film in a drum coater, using water and, relative to 100 parts by weight of the tablets,
H. 0.5 to 10 parts by weight film former
I. 0.0 to 2 parts by weight plasticizer
J. 0.005 to 2 parts by weight anti-adherent agent, and
K. dye and/or pigments.

14. Pharmaceutical composition which can be obtained according to the process according to one of claims 4 to 13.

15. Use of a pharmaceutical composition according to one of claims 1 to 2 and 14 for the preparation of a medicament for the prevention or the treatment of primarily generalised tonic-clonic seizures and/or focal seizures with or without secondary generalisation.

16. Use of a pharmaceutical composition according to one of claims 1 to 2 and 14 for the preparation of a medicament for the prevention or the treatment of neuralgic and cerebrovascular pains or for alcohol detoxification.

17. Use of a pharmaceutical composition according to one of claims 1 to 2 and 14 for the preparation of a medicament which is suitable for once-a-day administration.

18. Use of a pharmaceutical composition according to one of claims 1 to 2 and 14 for the preparation of a medicament which is suitable for once-a-day administration for the prevention or the treatment of neuralgic and cerebrovascular pains or for alcohol detoxification.

19. Use of a pharmaceutical composition according to one of claims 1 to 2 and 14 for the preparation of a medicament which is suitable for once-a-day administration for the prevention or the treatment of primarily generalised tonic-clonic seizures and/or focal seizures with or without secondary generalisation.

## Revendications

1. Composition pharmaceutique contenant de l'oxcarbazépine qui libère *in vitro* selon le procédé Paddle USP (USP 24, Méthode 724, App. 2 dans 11 de solution de dodécylsulfate de sodium à 2 % en tant que milieu de libération, à une vitesse d'agitation de 75 U/min) les quantités suivantes d'oxcarbazépine :
15 min : 55 à 85 %
30 min : 75 à 95 %
45 min : 85 à 100 %
60 min : 90 à 100 %.

2. Composition pharmaceutique selon la revendication 1, contenant de l'oxcarbazépine, qui libère *in vitro* selon le procédé Paddle USP (USP 24, Méthode 724, App. 2 dans 11 de solution de dodécylsulfate de sodium à 2 % en tant que milieu de libération, à une vitesse d'agitation de 75 U/min) les quantités suivantes d'oxcarbazépine :
15 min: 65 à 80 %
30 min : 85 à 95 %
45 min : 90 à 100 %
60 min : 95 à 100 %.

3. Procédé de production d'une composition pharmaceutique selon l'une quelconque des revendications précédentes, comprenant la préparation d'un mélange contenant, rapporté à son poids total :
a. 60 à 95 % en poids d'oxcarbazépine,
b. 3 à 30 % en poids de cellulose microcristalline,
c. 1 à 20 % en poids de copolymère d'ammonium-méthacrylate et/ou de polymère d'acide méthacrylique,
d. 0,05 à 4 % en poids d'un agent de délitement et
e. un colorant,
puis le compactage du mélange.

4. Procédé selon la revendication 3, comprenant la préparation d'un mélange qui contient, rapporté à son poids total :
a. 80 à 90 % en poids d'oxcarbazépine,
b. 5 à 15 % en poids de cellulose microcristalline,
c. 2 à 10 % en poids de copolymère d'ammonium-méthacrylate et/ou de polymère d'acide méthacrylique,
d. 0,1 à 2 % en poids d'un agent de délitement et
e. un colorant,
puis le compactage du mélange.

5. Procédé selon l'une quelconque des revendications 3 ou 4, dans lequel la masse compactée est tamisée et est conditionnée telle quelle ou éventuellement avec des adjuvants, dans des gélules ou des sachets.

6. Procédé selon l'une quelconque des revendications 3 ou 4, dans lequel on incorpore, après compactage, par rapport à 100 parties en poids de la masse compactée,
f. 0,2 à 5 parties en poids de stéarate de magnésium et
g. 10 à 50 parties en poids de cellulose microcristalline
et l'on transforme le mélange ainsi obtenu en comprimés.

7. Procédé de production d'une composition pharmaceutique selon l'une quelconque des revendications 1 à 2, dans lequel on prépare, en lit fluidisé ou dans un mélangeur rapide en ajoutant de l'eau, un granulat contenant, rapporté à son poids total,
A. 60 à 95 % en poids d'oxcarbazépine,
B. 3 à 30 % en poids de cellulose microcristalline,
C. 0,05 à 4 % en poids d'agent de délitement,
D. 1 à 20 % en poids de polymère
E. 0,2 à 5 % en poids de plastifiant,
F. 0 à 5 % en poids d'agent de décollement,
G. un colorant.

8. Procédé selon la revendication 5, dans lequel le granulat contient, rapporté à son poids total :
A. 80 à 90 % en poids d'oxcarbazépine,
B. 5 à 15 % en poids de cellulose microcristalline,
C. 0,1 à 2 % en poids d'agent de délitement,
D. 2 à 10 % en poids de polymère
E. 0,4 à 2,5 % en poids de plastifiant,
F. 0 à 2,5 % en poids d'agent de décollement,
G. un colorant q.s.

9. Procédé selon l'une quelconque des revendications 7 ou 8, dans lequel on incorpore par mélange, rapporté à 100 parties en poids de granulat,
H. 0,2 à 0,5 parties en poids de lubrifiant de comprimés et
I. 10 à 50 parties en poids de cellulose microcristalline, et l'on transforme le mélange ainsi obtenu en comprimés.

10. Procédé selon l'une quelconque des revendications 3 ou 4, dans lequel la masse compactée est pelliculée, dans un mélangeur rapide en présence d'eau,
en utilisant, rapporté à 100 parties en poids de masse compactée,
F. 0,5 à 10 parties en poids de copolymère d'acide méthacrylique
G. 0,025 à 2 parties en poids de plastifiant
H. 0,025 à 2 parties en poids d'agent de décollement.

11. Procédé selon la revendication 10, dans lequel on incorpore par mélange, rapporté à 100 parties en poids de la masse compacte pelliculée,
I. 0,2 à 0,5 parties en poids de lubrifiant de comprimé et
J. 10 à 50 parties en poids de cellulose microcristalline
et l'on transforme le mélange ainsi obtenu en comprimés.

12. Procédé selon la revendication 6, dans lequel les comprimés sont pelliculés dans un dispositif d'enrobage à tambour en présence d'eau et en utilisant, rapporté à 100 parties en poids de comprimés,
H. 0,5 à 10 parties en poids de copolymère d'acide méthacrylique,
I. 0,025 à 2 parties en poids de plastifiant,
J. 0,05 à 2 parties en poids d'agent de décollement et
K. un colorant et/ou des pigments.

13. Procédé selon la revendication 6, dans lequel les comprimés sont pelliculés dans un dispositif d'enrobage à tambour en présence d'eau et en utilisant, rapporté à 100 parties en poids de comprimés,
H. 0,5 à 10 parties en poids de copolymère d'acide méthacrylique,
I. 0,0 à 2 parties en poids de plastifiant,
J. 0,005 à 2 parties en poids d'agent de décollement et
K. un colorant et/ou des pigments.

14. Composition pharmaceutique susceptible d'être obtenue par le procédé selon l'une quelconque des revendications 4 à 13.

15. Utilisation d'une composition pharmaceutique selon l'une quelconque des revendications 1 à 2 et 14, pour la production d'un médicament destiné à la prévention et au traitement des crises tonico-cloniques primaires généralisées et/ou des crises focales avec ou sans généralisation secondaire.

16. Utilisation d'une composition pharmaceutique selon l'une quelconque des revendications 1 à 2 et 14, pour la production d'un médicament destiné à la prévention ou au traitement des douleurs névralgiques et cérébro-vasculaires ou au sevrage alcoolique.

17. Utilisation d'une composition pharmaceutique selon l'une quelconque des revendications 1 à 2 et 14 pour la production d'un médicament à prendre une fois par jour.

18. Utilisation d'une composition pharmaceutique selon l'une quelconque des revendications 1 à 2 et 14 pour la production d'un médicament à prendre une fois par jour pour la prévention ou le traitement des douleurs névralgiques et cérébro-vasculaires ou pour le sevrage alcoolique.

19. Utilisation d'une composition pharmaceutique selon l'une quelconque des revendications 1 à 2 et 14 pour la production d'un médicament à prendre une fois par jour pour la prévention ou le traitement des crises tonico-cloniques primaires généralisées et/ou des crises focales avec ou sans généralisation secondaire.
